# EUROPEAN PATENT APPLICATION

(11) **EP 4 582 182 A1**
(43) Date of publication of application: **09.07.2025**
(21) Application number: 23860537.2
(22) Date of filing: 01.09.2023
(51) Int. Cl.: B01J 31/22, B01J 31/06, C07C 51/00, C07C 53/02

(54) **DEHYDROGENATION/CARBON DIOXIDE HYDROGENATION CATALYST, AND HYDROGEN GAS PRODUCTION METHOD AND CARBON DIOXIDE HYDROGENATION METHOD EACH USING SAME**

(30) Priority: 02.09.2022 JP 2022139988
(71) Applicant: National Institute of Advanced Industrial Science and Technology, Chiyoda-ku Tokyo 100-8921 (JP)
(72) Inventor: KAWANAMI, Hajime, Tsukuba-shi, Ibaraki 305-8560 (JP)
(74) Representative: Gill Jennings & Every LLP
(86) International application number: PCT/JP2023/032059
(87) International publication number: WO 2024/048775

(57) **Abstract**

Provided is a dehydrogenation/carbon dioxide hydrogenation catalyst characterized by comprising an iridium complex represented by formula (3) and having a ligand bound to a polymer support. (In formula (3), pc represents a portion of the polymer support; m and n each independently represent a positive integer or 0; X and Y each independently represent a positive integer of 1 to 6; R1's each independently represent a hydrogen atom or a methyl group; R2's each independently represent a hydrogen atom or a methyl group; P represents a molecule or an ion of any one of H₂O, chlorine (Cl), bromine (Br) and acetonitrile (CH₃CN); and C represents any one of a hydroxide ion (OH⁻), a sulfate ion (SO₄²⁻), a chlorine ion (Cl⁻), a bromine ion (Br⁻), a nitrate ion (NO₃⁻) and a trifluoromethanesulfonate ion (CF₃SO₃⁻).)

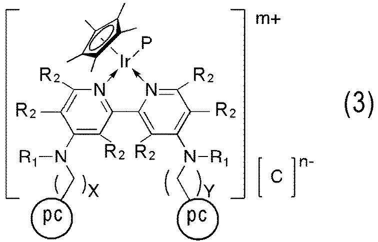

## Description

### [Technical Field]

The present invention relates to a dehydrogenation/carbon dioxide hydrogenation catalyst that generates formic acid or the like from carbon dioxide and hydrogen or selectively decomposes formic acid or the like to generate carbon dioxide and hydrogen, and a hydrogen gas production method and a carbon dioxide hydrogenation method each using the same.

### [Background Art]

Hydrogen has drawn attention as a next-generation energy source, and various technologies for storing, transporting, and utilizing hydrogen are being developed. Herein, for example, there are attempts to convert electrical energy into hydrogen, and then convert the hydrogen into other materials (hydrogen carriers) for storage, transport, and the like. As such hydrogen carriers, formic acid has been proposed together with liquid hydrogen, ammonia, methylcyclohexane, or the like.

Formic acid can be catalytically and selectively decomposed to give carbon dioxide and hydrogen, and conversely, formic acid can be catalytically synthesized from hydrogen and carbon dioxide. There are a number of catalysts available for a dehydrogenation reaction from formic acid and a formic acid generation reaction by hydrogenation from carbon dioxide. However, homogeneous catalysts containing complexes are said to have a higher formic acid decomposition rate or higher formic acid generation rate per catalyst than those in heterogeneous catalysts containing solids, such as noble metals.

As the homogeneous catalysts usable for the decomposition and the synthesis of formic acid, ruthenium complexes described in Patent Document 1, for example, are known.

### [Citation List]

### [Patent Document]

[Patent Document 1] JP 2019-218579 A

### [Summary of Invention]

### [Technical Problem]

For practical purposes, the decomposition and the synthesis of formic acid are preferably performed not in a batch manner but by a continuous reaction in a flow manner. The homogeneous catalyst containing a metal complex is required to be immobilized on an inorganic support or a polymer support to be formed into an immobilized catalyst. In general, the homogenous catalyst can be supported on a support, such as silica or a polymer, by forming a bond with a ligand of a metal complex serving as the catalyst.

In the immobilized catalyst where the metal complex is immobilized on the support, the metal complex is eluted by about 20 to 30% in each reaction. This significantly reduces the catalytic activity to half or less after several uses. Therefore, a supported catalyst for formic acid formation/decomposition has been demanded which enables efficient decomposition and synthesis of formic acid even in the flow manner.

The present invention has been made in view of the above-described circumstances. It is an object of the present invention to provide a dehydrogenation/carbon dioxide hydrogenation catalyst suitable for the decomposition and the synthesis of formic acid or the like in the flow manner, and a hydrogen gas production method and carbon dioxide hydrogenation method each using the same.

### [Solution to Problem]

The present invention has proposed an immobilized catalyst structured so that an amino group or a hydroxyl group is used for a ligand of a metal complex as a homogeneous complex catalyst and the ligand is incorporated into a polymer main chain and further an immobilized catalyst structured so that an amino group or a hydroxyl group is used for a ligand of a metal complex as a homogeneous complex catalyst and the ligand is incorporated into a polymer side chain. The immobilized catalyst functions as a heterogeneous catalyst while maintaining high activity of the homogeneous complex catalyst and enables a continuous reaction in a flow manner in a dehydrogenation reaction from formic acid and a formic acid and formate generation reaction from hydrogen and carbon dioxide. At the same time, the recovery of the catalyst after the reaction can be facilitated. Such a catalyst can be effectively used not only in the flow manner but in the batch manner.

A dehydrogenation/carbon dioxide hydrogenation catalyst according to the present invention contains an iridium complex represented by Formula (3) below which has a ligand bound to a polymer support, 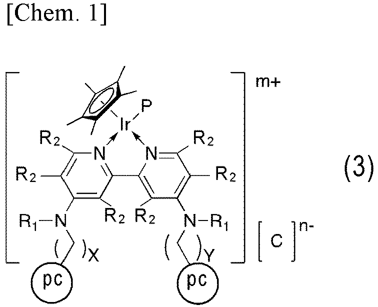 (In Formula (3) above, pc each represents a part of the polymer support, m and n each independently represent a positive integer or 0, X and Y each independently represent a positive integer from 1 to 6, R₁s each independently represent hydrogen or a methyl group, R₂s each independently represent hydrogen or a methyl group, P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).).

In the above-described invention, the dehydrogenation/carbon dioxide hydrogenation catalyst may further contain a polymer support represented by Formula (1) below, 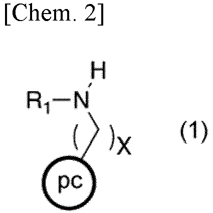 (In Formula (1) above, pc represents a part of the polymer support, X represents a positive integer from 1 to 6, and R₁ each independently represents hydrogen or a methyl group.).

In the above-described invention, the dehydrogenation/carbon dioxide hydrogenation catalyst may further contain a ligand represented by Formula (2) below bound to a polymer support, 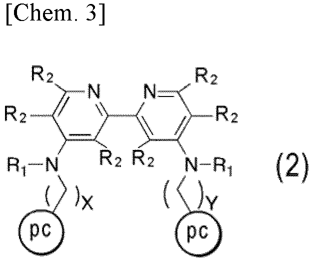 (In Formula (2) above, pc each represents a part of the polymer support, X and Y each independently represent a positive integer from 1 to 6, R₁s each independently represent hydrogen or a methyl group, and R₂s each independently represent hydrogen or a methyl group.).

In the above-described invention, the polymer support represented by Formula (1) above may be polyethyleneimine, polystyrene, or a copolymer of ethyleneimine and styrene.

The dehydrogenation/carbon dioxide hydrogenation catalyst according to the present invention contains an iridium complex which has a ligand represented by Formula (6) below bound to a polymer support, 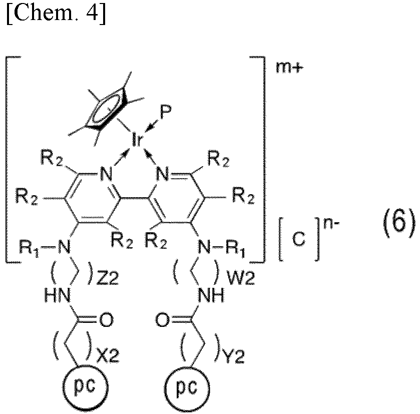 (In Formula (6) above, pc each represents a part of the polymer support, m and n each independently represent a positive integer or 0, X2 and Y2 each independently represent an integer from 0 to 6, Z2 and W2 each independently represent a positive integer from 1 to 6, R₁s each independently represent hydrogen or a methyl group, R₂s each independently represent hydrogen or a methyl group, P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).).

In the above-described invention, the dehydrogenation/carbon dioxide hydrogenation catalyst may further contain a polymer support represented by Formula (4) below, 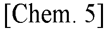 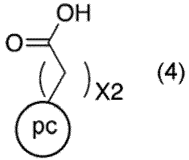 (In Formula (4) above, pc represents a part of the polymer support and X2 represents an integer from 0 to 6.).

In the above-described invention, the dehydrogenation/carbon dioxide hydrogenation catalyst may further contain a ligand represented by Formula (5) below bound to a polymer support, 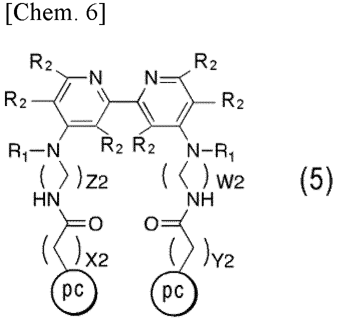 (In Formula (5) above, pc each represents a part of the polymer support, X2 and Y2 each independently represent an integer from 0 to 6, Z2 and W2 each independently represent a positive integer from 1 to 6, R₁s each independently represent hydrogen or a methyl group, and R₂s each independently represent hydrogen or a methyl group.)

In the above-described invention, the polymer support represented by Formula (4) above may be polyacrylic acid, polymethacrylic acid, polymaleic acid, or a copolymer of at least two selected from the group consisting of acrylic acid, methacrylic acid, and maleic acid.

A hydrogen gas production method according to the present invention includes generating a gas containing hydrogen and carbon dioxide and having a total pressure of 0.1 MPa or more and 200 MPa or less from a solution containing formic acid, in which the catalyst described above is used.

A carbon dioxide hydrogenation method according to the present invention includes generating formic acid or formate from a gas containing hydrogen and carbon dioxide and having a total pressure of 0.1 MPa or more and 200 MPa or less, in which the above-described catalyst is used.

### [Brief Description of the Drawings]

[Fig. 1] FIG. 1 is a graph showing the results of the generated gas amount according to the present invention.
[Fig. 2] FIG. 2 is a graph showing the results of the turnover frequency with respect to the number of repetitions of a catalyst according to the present invention.
[Fig. 3] FIG. 3 is a graph showing the results of measuring the initial formic acid decomposition rate to the ratio between the iridium support weight and the PEI weight according to the present invention.
[Fig. 4] FIG. 4 is a graph showing the results of the generated gas amount according to the present invention.
[Fig. 5] FIG. 5 is a graph showing the results of the catalyst's turnover number with respect to a reaction time according to the present invention.
[Fig. 6] FIG. 6 is a graph showing the results of the catalyst turnover number with respect to a reaction time according to the present invention.
[Fig. 7] FIG. 7 is a graph showing the results of Example 7.
[Fig. 8] FIG. 8 is a graph showing the results of Example 7.
[Fig. 9] FIG. 9 is a graph showing the results of Example 8.
[Fig. 10] FIG. 10 is a graph showing the results of Reference Example.
[Fig. 11] FIG. 11 is an explanatory view illustrating a batch-type hydrogen production test.
[Fig. 12] FIG. 12 is a graph showing the results of Example 14.
[Fig. 13] FIG. 13 is an explanatory view illustrating a flow-type hydrogen production test.
[Fig. 14] FIG. 14 is a graph showing the results of Example 15.

### [Description of Embodiments]

Hereinafter, representative examples of the present invention are described.

A dehydrogenation/carbon dioxide hydrogenation catalyst according to one aspect of the present invention contains an iridium complex represented by Formula (3) below which has a ligand bound to a polymer support. Herein, in Formula (3) below, pc each represents a part of the polymer support, m and n each independently represent a positive integer or 0 (preferably 1 to 3, more preferably 1 to 2, and still more preferably 1), X and Y each independently represent a positive integer from 1 to 6 (preferably 1 to 4 and more preferably 1 to 2), R₁s each independently represent hydrogen or a methyl group (preferably hydrogen), R₂s each independently represent hydrogen or a methyl group (preferably hydrogen), P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).

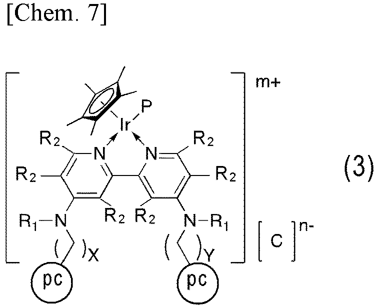

Herein, the iridium complex represented by Formula (3) above contains a salt of the iridium complex represented by Formula (3). For example, when m and n represent 1, the iridium complex represented by Formula (3) is a salt of the iridium complex having C as a counter anion. The binding site of the polymer support is not particularly limited, and may be the terminal of a main chain of a polymer constituting the polymer support or may be a side chain of the polymer. pc in Formula (3) each may represent a part of the same polymer support or may represent a part of different polymer supports. The fact that pc each represents a part of different polymer supports indicates a structure in which the different polymer supports are crosslinked. This makes it easy for the polymer supports to form a gel or a solid.

The ligand bound to the polymer support refers to, for example, a portion equivalent to Formula (2) below in Formula (3) above.

The inclusion of the iridium complex represented by Formula (3) above enables the action as the dehydrogenation/carbon dioxide hydrogenation catalyst.

The content ratio of the iridium complex represented by Formula (3) above in the dehydrogenation/carbon dioxide hydrogenation catalyst is not particularly limited, and can be set to 0.04 to 0.10% by weight.

The dehydrogenation/carbon dioxide hydrogenation catalyst preferably further contains a polymer support represented by Formula (1) below. By the inclusion of the polymer support represented by Formula (1) below, the optimal acidity/basicity in a formic acid decomposition or generation reaction can be held, and high activity is easily maintained over a long period of time. 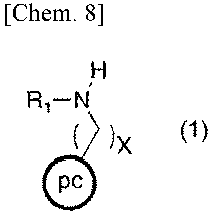 (In Formula (1) above, pc represents a part of the polymer support, X represents a positive integer from 1 to 6 (preferably 1 to 4 and more preferably 1 to 2), and R₁s each independently represent hydrogen or a methyl group (preferably hydrogen).)

The content ratio of the polymer support represented by Formula (1) above in the dehydrogenation/carbon dioxide hydrogenation catalyst is not particularly limited, and can be set to 50 to 99% by weight.

The value of the weight ratio of the content of the polymer support (e.g., polyethyleneimine) represented by Formula (1) above to the iridium content in the dehydrogenation/carbon dioxide hydrogenation catalyst is not particularly limited, and is preferably 25 to 500, more preferably 50 to 450, and still more preferably 75 to 300.

The dehydrogenation/carbon dioxide hydrogenation catalyst preferably further contains a ligand represented by Formula (2) below bound to the polymer support. The ligand represented by Formula (2) below is covalently bound to the polymer support via a nitrogen atom. The binding site of the polymer support is not particularly limited and may be the terminal of a main chain of a polymer constituting the polymer support or may be a side chain of the polymer. pc each in Formula (2) may represent a part of the same polymer support or a part of different polymer supports. The fact that pc each represents a part of different polymer supports indicates a structure in which the different polymer supports are crosslinked. This makes it easy for the polymer supports to form a gel or a solid.

Due to the fact that the ligand represented by Formula (2) below is contained, the ligand can capture the iridium complex when the iridium complex is eluted in the reaction to suppress the elution of the iridium. As a result, the life of the catalyst is likely to be prolonged. 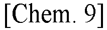 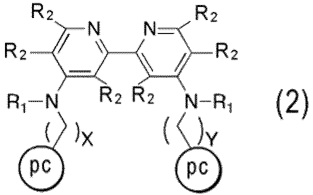 (In Formula (2) above, pc each represents a part of the polymer support, X and Y each independently represent a positive integer from 1 to 6 (preferably 1 to 4 and more preferably 1 to 2), R₁s each independently represent hydrogen or a methyl group (preferably hydrogen), and R₂s each independently represent hydrogen or a methyl group (preferably hydrogen).)

The content ratio of the ligand represented by Formula (2) above bound to the polymer support in the dehydrogenation/carbon dioxide hydrogenation catalyst is not particularly limited, and can be set to 1 to 50% by weight.

A dehydrogenation/carbon dioxide hydrogenation catalyst according to one aspect of the present invention contains an iridium complex represented by Formula (6) below which has a ligand bound to a polymer support. Herein, in Formula (6) below, pc each represents a part of the polymer support, m and n each independently represent a positive integer or 0 (preferably 1 to 3, more preferably 1 to 2, and still more preferably 1), X2 and Y2 each independently represent an integer from 0 to 6 (preferably 1 to 6, more preferably 1 to 4, and still more preferably 1 to 2), Z2 and W2 each independently represent a positive integer from 1 to 6 (preferably 1 to 4 and more preferably 1 to 2), R₁s each independently represent hydrogen or a methyl group (preferably hydrogen), R₂s each independently represent hydrogen or a methyl group (preferably hydrogen), P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).

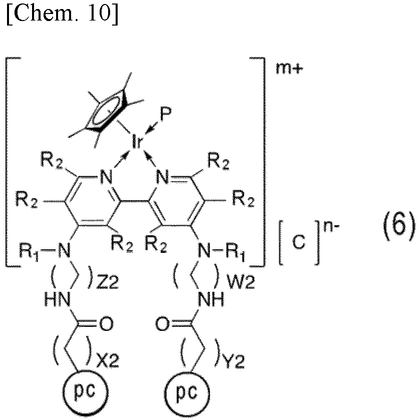

Herein, the iridium complex represented by Formula (6) above contains a salt of the iridium complex represented by Formula (6). For example, when m and n represent 1, the iridium complex represented by Formula (6) is a salt of the iridium complex having C as a counter anion. The binding site of the polymer support is not particularly limited, and may be the terminal of a main chain of a polymer constituting the polymer support or may be a side chain of the polymer. pc in Formula (6) each may represent a part of the same polymer support or may represent a part of different polymer supports. The fact that pc each represents a part of different polymer supports indicates a structure in which the different polymer supports are crosslinked. This makes it easy for the polymer supports to form a gel or a solid.

The ligand bound to the polymer support refers to, for example, a portion equivalent to Formula (5) below in Formula (6) above.

The inclusion of the iridium complex represented by Formula (6) above enables the action as the dehydrogenation/carbon dioxide hydrogenation catalyst.

The content ratio of the iridium complex represented by Formula (6) above in the dehydrogenation/carbon dioxide hydrogenation catalyst is not particularly limited, and can be set to 0.04 to 0.10% by weight.

The dehydrogenation/carbon dioxide hydrogenation catalyst preferably further contains a polymer support represented by Formula (4) below. By the inclusion of the polymer support represented by Formula (4) below, the optimal acidity/basicity in a formic acid decomposition or generation reaction can be held, and high activity is easily maintained over a long period of time. 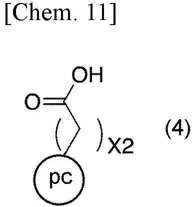 (In Formula (4) above, pc represents a part of the polymer support and X2 represents an integer from 0 to 6 (preferably 1 to 6, more preferably 1 to 4, and still more preferably 1 to 2).)

The content ratio of the polymer support represented by Formula (4) above in the dehydrogenation/carbon dioxide hydrogenation catalyst is not particularly limited, and can be set to 1 to 50% by weight.

The value of the ratio of the content of the polymer support (e.g., polyacrylic acid) represented by Formula (4) above to the iridium content in the dehydrogenation/carbon dioxide hydrogenation catalyst is not particularly limited, and is preferably 25 to 500, more preferably 50 to 450, and still more preferably 75 to 300.

The dehydrogenation/carbon dioxide hydrogenation catalyst preferably further contains a ligand represented by Formula (5) below bound to a polymer support. The ligand represented by Formula (5) below is covalently bound to the polymer support via an amide bond. The binding site of the polymer support is not particularly limited and may be the terminal of a main chain of a polymer constituting the polymer support or may be a side chain of the polymer. pc each in Formula (5) may represent a part of the same polymer support or a part of different polymer supports. The fact that pc each represents a part of different polymer supports indicates a structure in which the different polymer supports are crosslinked. This makes it easy for the polymer supports to form a gel or a solid.

Due to the fact that the ligand represented by Formula (5) below is contained, the ligand can capture the iridium complex, when the iridium complex is eluted in the reaction, to suppress the elution of the iridium. As a result, the life of the catalyst is likely to be prolonged. 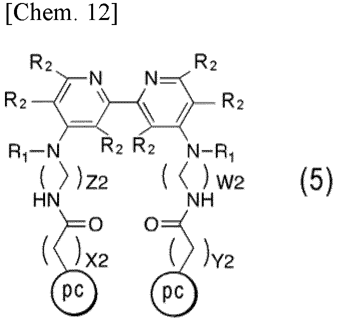 (In Formula (5) above, pc each represents a part of the polymer support, X2 and Y2 each independently represent an integer from 0 to 6 (preferably 1 to 6, more preferably 1 to 4, and still more preferably 1 to 2), Z2 and W2 each independently represent a positive integer from 1 to 6 (preferably 1 to 4 and more preferably 1 to 2), R₁s each independently represent hydrogen or a methyl group (preferably hydrogen), and R₂s each independently represent hydrogen or a methyl group (preferably hydrogen).)

The content ratio of the ligand represented by Formula (5) above bound to the polymer support in the dehydrogenation/carbon dioxide hydrogenation catalyst is not particularly limited, and can be set to 1 to 50% by weight.

The use of the above-described catalyst enables the generation of a gas containing hydrogen and carbon dioxide and having a total pressure of 0.1 MPa or more and 200 MPa or less typically from a solution containing formic acid. At this time, the gas can be generated in a batch manner and also can be generated in a flow manner.

Further, from the gas containing hydrogen and carbon dioxide and having a total pressure of 0.1 MPa or more and 200 MPa or less, formic acid or formate can be generated. At this time, the formic acid or the formate can be generated in a batch manner and also can be generated in a flow manner.

### [Polymer support]

As described above, the polymer support used in a supported complex catalyst (dehydrogenation/carbon dioxide hydrogenation catalyst) is represented by Formula (1) or Formula (4) above and has the function of holding the acid/base concentration optimal in the decomposition/generation of formic acid. Therefore, affinity with water is also important. Examples include polyalkylenimines and polyalkylenecarboxylic acids, and specifically polyethyleneimine, polybutyleneimine, polyacrylic acid, polymethacrylic acid, polymaleic acid, and the like are suitably usable. Further, copolymers containing the polyalkylenimines and the polyalkylenecarboxylic acids are also suitably usable.

Other polymer supports are also usable, and, for example, polystyrene and a copolymer of the above polymer and polystyrene, such as a copolymer of ethyleneimine and styrene, are also usable. Further, a copolymer of at least two selected from the group consisting of acrylic acid, methacrylic acid, and maleic acid is also usable.

### [Metal complex]

The central metal used in the supported complex catalyst can constitute a catalyst using one or more types of iron, cobalt, nickel, ruthenium, rhodium, palladium, osmium, iridium, platinum of Group VIII, copper, silver, and gold of Group IB, and zinc, cadmium, and mercury of Group IIB in the short-period periodic table. One or more types of iron, cobalt, nickel, ruthenium, rhodium, palladium, osmium, iridium, and platinum of Group VIII are preferable, one or more types of nickel, ruthenium, rhodium, palladium, iridium, and platinum are more preferable, and one or more types of ruthenium, rhodium, palladium, and iridium are most preferable. Preferably, metal particles are usable.

### [Concentration of formic acid]

The present catalyst can efficiently produce hydrogen and carbon dioxide from formic acid even when the formic acid has a low concentration. Therefore, although the concentration range is not particularly limited, the use of formic acid having a higher concentration is more efficient, because more hydrogen and carbon dioxide can be extracted from the formic acid per unit volume. However, economically, formic acid having a low concentration is available at a low cost and is not classified as a hazardous substance, and therefore any particular safety measure in accordance with the Fire Service Act is not required. Therefore, on the high concentration side, an aqueous formic acid solution having a concentration of 78% by mass or less is preferable, and hydrogen and carbon dioxide can be efficiently produced from formic acid by the use of more suitably an aqueous formic acid solution having a concentration of 85% by mass or less, still more preferably an aqueous formic acid solution having a concentration of 90% by mass or less, and most preferably an aqueous formic acid solution having a concentration of 99.8% by mass or less. The lower limit of the concentration range is not particularly limited, and an aqueous formic acid solution having a concentration of 0.1% by mass or more is preferable, and hydrogen and carbon dioxide can be efficiently produced from formic acid by the use of more suitably an aqueous formic acid solution having a concentration of 1% by mass or more and most preferably an aqueous formic acid solution having a concentration of 5% by mass or more. From the above, the formic acid concentration is not particularly limited, and can be set to, for example, 1% by mass to 78% by mass, preferably 3 to 78% by mass, 5 to 85% by mass, 5 to 90% by mass, and 5 to 99.8% by mass.

### [Formic acid purity]

With the present catalyst, the reaction can be suitably performed also with a mixed solution obtained by adding other compounds to the aqueous formic acid solution. Specific examples of the other compounds include methanol, ethanol, propanol, isopropanol, diethylether, tetrahydrofuran, dioxane, acetone, methyl ethyl ketone, acetic acid, propionic acid, dimethylformamide, diethylformamide, dimethylsulfoxide, chloroform, dichloromethane, trichloroethane, hexane, pentane, and the like. Even when these compounds are contained, hydrogen and carbon dioxide can be suitably produced from formic acid.

However, the presence of the compounds other than formic acid sometimes reduce the performance, and therefore the concentrations of the following compounds that can be contained in the aqueous solution of formic acid are preferably set to concentrations equal to or less than a predetermined concentration. More specifically, the reaction can be suitably performed by setting the concentration of each of fluorine (fluorine ion), chlorine (or chlorine ion), bromine (bromine ion), and iodine (iodine ion) to 1 ppm by mass or less.

### [Gas pressure range]

The pressures of hydrogen and carbon dioxide are not particularly limited, and, for example, the reaction can be performed at a total pressure of 0.1 MPa or higher, with hydrogen at 0.05 MPa or more and carbon dioxide at 0.05 MPa or more. The total pressure of hydrogen and carbon dioxide is preferably 1 MPa or more, and the total pressure is more preferably 5 MPa or more and still more preferably 8 MPa or more. The total pressure can be set to 1 to 200 MPa, 5 to 200 MPa, and 8 to 200 MPa, for example. When gases other than hydrogen and carbon dioxide are contained, the pressures thereof are not particularly limited and can be set as appropriate.

### [Ratio between hydrogen and carbon dioxide]

As the ratio between hydrogen and carbon dioxide, hydrogen and carbon dioxide are preferably used in a proper ratio, because too much hydrogen sometimes results in the formation of methane or methanol. However, carbon dioxide even in a large excess can also act as a solvent, and therefore the ratio of carbon dioxide to hydrogen is not particularly limited. Hydrogen is present in the range of preferably 10 ppm by mass or more (0.001% by mass) to 50% by mass or less with respect to carbon dioxide, and, in terms of efficiency, is used preferably in the range of 1% by mass or more to 50% by mass or less, more preferably in the range of 10% by mass or more to 50% by mass or less, and still more preferably in the range of 20% by mass or more to 50% by mass or less.

### [Gas purity]

As the gases other than hydrogen and carbon dioxide, any gas inert to the reaction is usable as appropriate and, for example helium, nitrogen, argon, xenon, methane, ethane, and propane are usable. Further, these gases are usable as appropriate in the range of 10 ppm by mass (0.001% by mass) or more and 50% by mass or less with respect to hydrogen and carbon dioxide. In terms of efficiency, the lower concentration is better, and these gases are usable preferably in the range of 20% by mass or less, more preferably in the range of 10% by mass or less, and still more preferably in the range of 1% by mass or less. For example, the range can be set to 10 ppm by mass or more and 20% by mass or less, 10 ppm by mass or more and 10% by mass or less, and 10 ppm by mass or more and 1% by mass or less.

### [Reaction temperature]

As the reaction temperature when formic acid is obtained from hydrogen and carbon dioxide, a gas containing a mixture of hydrogen and carbon dioxide can be suitably used in which the temperature is equal to or higher than particularly the critical temperature. The reaction can be performed by adjusting the reaction temperature as appropriate. Therefore, although the reaction temperature varies, the reaction can be performed by adjusting the reaction temperature as appropriate in the range of 0°C or more and 150°C or less, for example, and the reaction can be performed in the range of more suitably 10°C or more and 150°C or less, still more suitably 10°C or more and 100°C or less, and most suitably 50°C or more and 100°C or less.

### [Reaction time]

The reaction time when formic acid is obtained from hydrogen and carbon dioxide is not particularly limited, and can be adjusted as appropriate according to the purpose, because the reaction rate varies depending on each substance. In either case where the reaction is performed in the batch manner or the flow manner, the target substance can be obtained by adjusting the reaction time in the range of 1 minute or more and 72 hours or less, for example, and the compound to be targeted can be obtained by adjusting the reaction time in the range of suitably 10 minutes or more and 72 hours or less, more suitably 30 minutes or more and 48 hours or less, and most suitably 1 hour or more and 24 hours or less.

### [Reactor]

A reactor can be a flow-type reactor, and a batch-type reactor is also usable.

As described above, in the dehydrogenation reaction from formic acid and the formic acid and formate generation reaction from hydrogen and carbon dioxide, the immobilized catalyst can be used which contains a ligand having an amino group and further the iridium complex having an amino group with a polymer having an amino group or a carboxylic acid as a functional group as the support. In the conventional methods, the iridium complex is eluted in the reaction and the life of the catalyst is extremely short, while, when such an immobilized catalyst is used, the iridium complex eluted in the reaction is captured by another ligand and the elution of the iridium can be significantly reduced. Further, the functional group of the polymer support can hold the optimal acidity/basicity in the decomposition or formation reaction of formic acid, making it easy to maintain high activity over a longer period of time. Therefore, in the construction of a future hydrogen society, the use of the catalyst achieves the construction of a system containing formic acid as a hydrogen carrier.

### [EXAMPLES]

Hereinafter, Examples of the present invention are specifically described, but the present invention is not limited thereto. The determination of the conversion rate and the yield of the reaction is described later.

### [Example 1: Synthesis of immobilized catalyst]

To 1.06 g of polyethyleneimine (PEI, branched, Mw up to 25,000, Mn up to 10,000), 4,4'-dichloro-2,2'-bipyridine (0.32 g, 1.43 mmol) and water (1.02 mL) were added, followed by heating at 130°C for 24 hours. The obtained pale yellow solid was washed with chloroform, and then washed with water until no chlorine was detected, yielding 1.36 g of pale yellow solid. The solid (0.6 g) was dispersed in water to dissolve triaqua(pentamethylcyclopentadienyl)iridium(III) sulfate (11.6 mg, 13.0 µmol), followed by stirring at room temperature for 3 days, so that iridium was supported on the resultant substance. Then, the obtained solid was separated by a centrifuge, followed by drying while pressure was reduced, so that an immobilized catalyst (Cp*IrBPY/PEI, Formula (A), 0.6 g) was synthesized. 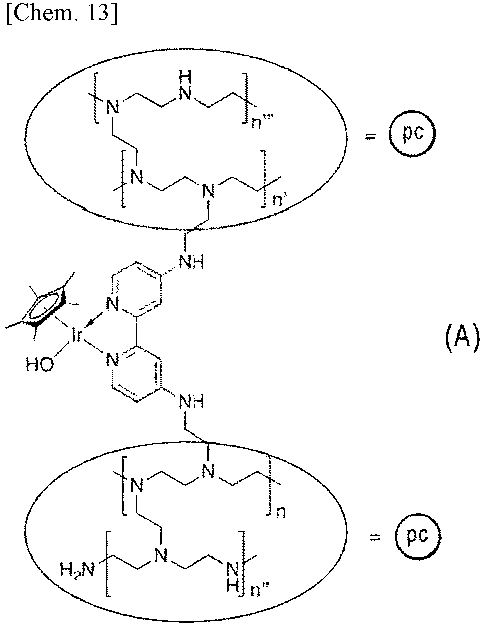 In Formula (A), n, n', and n‴ represent the degree of polymerization.

### [Example 2: Formic acid decomposition performance evaluation of immobilized catalyst]

In a 30 mL stainless steel autoclave, a magnetic stirrer and 13.5 mg of the immobilized catalyst (Cp*IrBPY/PEI, iridium content of 3.2 wt%) were placed, and then 17.5 mL of water and formic acid (98% or more, 2.5 mL) were placed, followed by heating to 80°C in a water bath. The generated gas was measured for the generation rate with a wet gas meter. After the gas generation was completed, formic acid (98% or more, 1 mL) was further added, and the measurement of the generation rate was repeated. The generated gas was collected in a sampling bag, and was measured for the composition by a gas chromatograph.

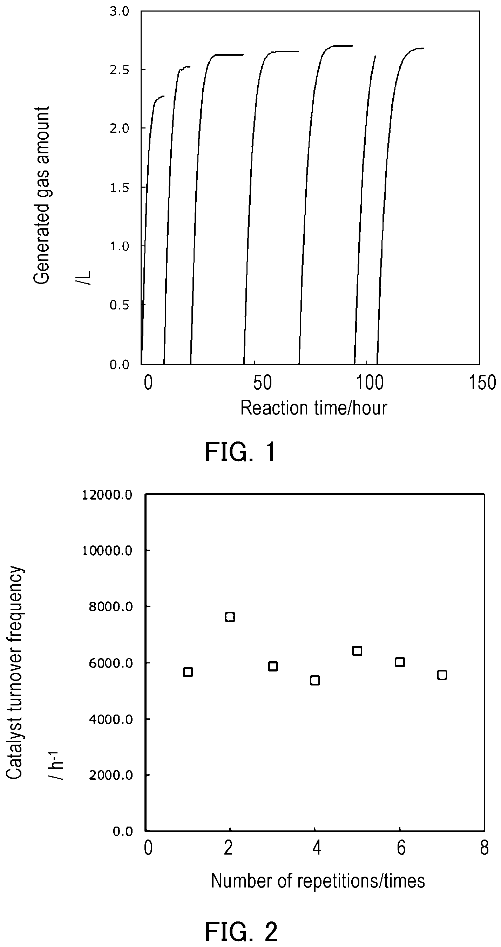

As illustrated in FIG. 1, the generation amount was slightly small only in the first time, but, from the second time onwards, the gas generation of about 2.6 L was detected, and almost 100% of formic acid was decomposed to be converted into carbon dioxide and hydrogen.

As illustrated in FIG. 2, the number of formic acid molecules catalyzed by iridium metal per hour was generally about 7000 h⁻¹. Even after seven repetitions, a decrease in the catalyst turnover frequency was hardly observed. When the composition of the generated gas was examined by a gas chromatograph, 50% of hydrogen and 50% of carbon dioxide were detected and no carbon monoxide was detected.

### [Example 3]

An immobilized catalyst (Cp*IrBPY/PEI) was obtained in the same manner as in Example 1, except that the addition amounts of the raw materials were changed so that the iridium content was 2.5 wt%, 5.5 wt%, 10 wt%, 25 wt%, or 50 wt%.

In a 30 mL stainless steel autoclave, a magnetic stirrer and the immobilized catalyst (Cp*IrBPY/PEI) having an iridium content different in predetermined amount were placed, and then 17.5 mL of water and formic acid (98% or more, 2.5 mL) were placed, followed by heating to 80°C in a water bath. The generated gas was measured for the generation rate with a wet gas meter. The generated gas was collected in a sampling bag, and was measured for the composition by a gas chromatograph.

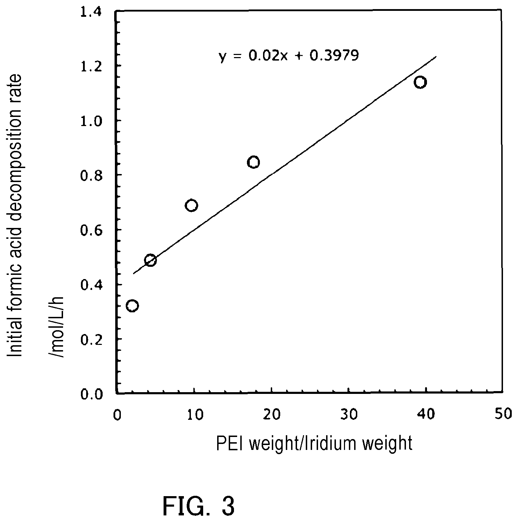

FIG. 3 shows the results of measuring the initial formic acid decomposition rate using the immobilized catalyst changed in the value of the ratio of the PEI (polyethyleneimine) weight to the Ir support weight using the above-described technique. It was found that the initial formic acid decomposition (dehydrogenation) rate increases with an increase in a ratio of the PEI weight to the iridium support weight. When the composition of the generated gas was examined by a gas chromatograph, 50% of hydrogen and 50% of carbon dioxide were detected and no carbon monoxide was detected.

### [Example 4]

In a 30 mL stainless steel autoclave, the immobilized catalyst (Cp*IrBPY/PEI) having a predetermined amount of iridium content obtained in the experiment of Example 1 was placed, and then 17.5 mL of water and formic acid (98% or more, 2.5 mL) were placed, followed by heating to 80°C in a water bath.

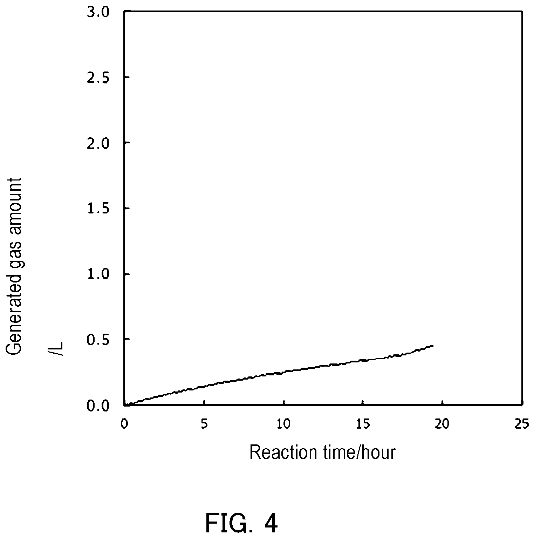

When the amount of iridium contained in the aqueous solution after the seventh reaction was examined by a high-frequency inductively coupled plasma emission spectrometry, the elution of 0.74% by mass of iridium was confirmed. This showed that iridium was hardly eluted. The solution containing the eluted iridium was used to examine the catalytic activity. FIG. 4 illustrates the results.

As illustrated in FIG. 4, the gas generation from formic acid was observed. The catalyst turnover frequency (TOF) was 6000 h⁻¹ or more, which showed that the active center iridium complex structure was maintained. More specifically, it was assumed that iridium alone was not eluted, but a part of the polyethyleneimine backbone was cleaved by hydrogen so that the iridium complex structure was eluted. The generated gas was collected in a sampling bag and measured for the composition by a gas chromatograph, so that no carbon monoxide was detected. Although the dehydrogenation reaction from formic acid was performed without the catalyst under similar conditions, the generated gas amount was equal to or less than the detection limit in a gas meter.

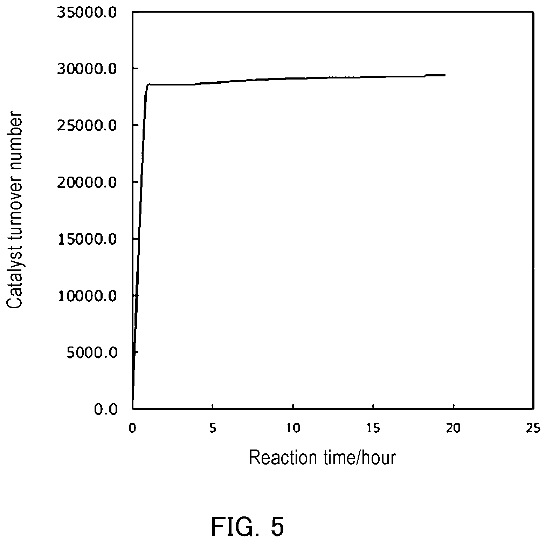

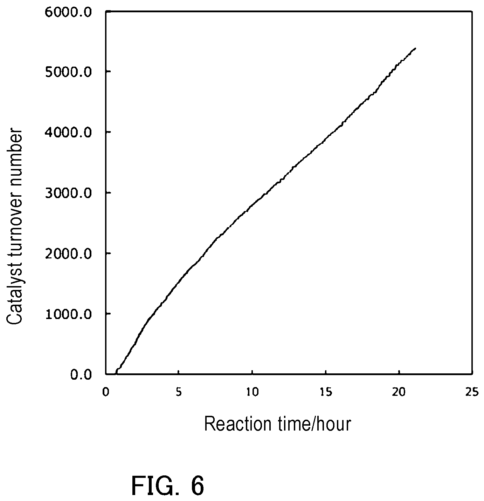

FIGS. 5 and 6 illustrate the catalyst turnover frequency with respect to the reaction time when the catalyst represented by Formula (A) was used and when the catalyst represented by Formula (B) was used, respectively. As a result, the catalyst represented by Formula (A) having the structure of being supported on PEI as the polymer support exhibited a high TOF value, while the catalyst represented by Formula (B) not having the structure of being supported on PEI as the polymer support exhibited a low TOF value. Also from this fact, it was inferred that a substance eluted into the aqueous solution in Example 4 was a slight amount of the active catalyst and that the structure of the iridium complex supported on PEI is the catalyst having the structure in which the PEI backbone was decomposed and eluted from the immobilized catalyst (Cp*IrBPY/PEI).

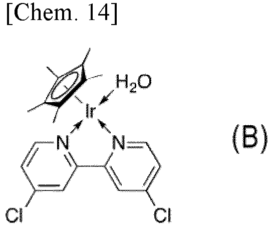

### [Example 5: High-pressure gas generation performance evaluation of immobilized catalyst]

In a 30 mL stainless steel autoclave, a magnetic stirrer and 13.5 mg of the immobilized catalyst (Cp*IrBPY/PEI, iridium content of 3.2 wt%) were placed, and then 17.5 mL of water and formic acid (98% or more, 2.5 mL) were placed, followed by heating to 80°C in a water bath. The pressure rise rate due to the generated gas was measured by a pressure sensor. After the pressure rise reached a steady state, the decomposition rate of formic acid was measured by a liquid chromatography, so that the decomposition rate was 90% by mass. After the gas formation was completed, formic acid (98% or more, 1 mL) was further added and the measurement of the generation rate was repeated. The generated gas was collected in a sampling bag, and was measured for the composition by a gas chromatograph.

The result of the implementation using the above-described technique showed that a 60 MPa pressure was generated. When the composition of the generated gas was examined by a gas chromatograph, 50% by mass hydrogen and 50% by mass carbon dioxide were detected, and no carbon monoxide was detected.

### [Example 6: High-pressure gas generation repetition performance evaluation of immobilized catalyst]

A high-pressure gas was generated by the same technique as that in Example 5. After the reaction was completed, 2.5 mL of formic acid was added, and a pressure rise test was performed again. As a result, the pressure rise rate was not changed even after the test was repeated three times. The formic acid decomposition rate was constant at 90% by mass in each test. When the collected generated gas was examined after the reaction, no carbon monoxide was detected. Further, after the three reactions were completed, when the amount of iridium contained in the reaction solution from which the immobilized catalyst was separated by filtration was examined, 0.42% by mass of iridium was eluted. Further, when the dehydrogenation reaction of formic acid was examined using this eluted solution, the TOF value was about 5000 h⁻¹.

The above results showed that the catalyst exhibits stable activity even under a high pressure of 50 MPa or more. It was proved that the catalyst decomposition was also suppressed, and, although slight elution was observed, the stability was not changed.

### [Example 7]

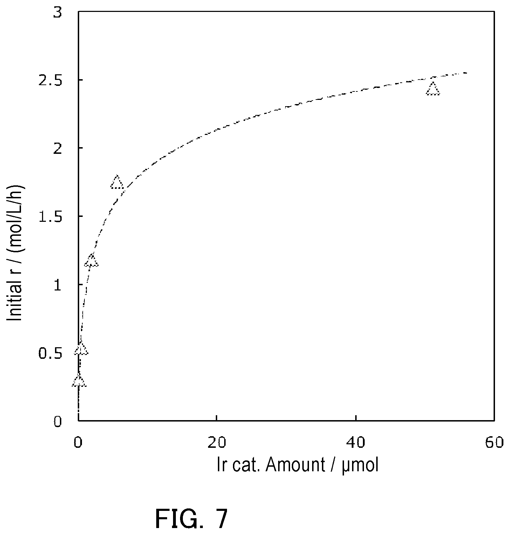

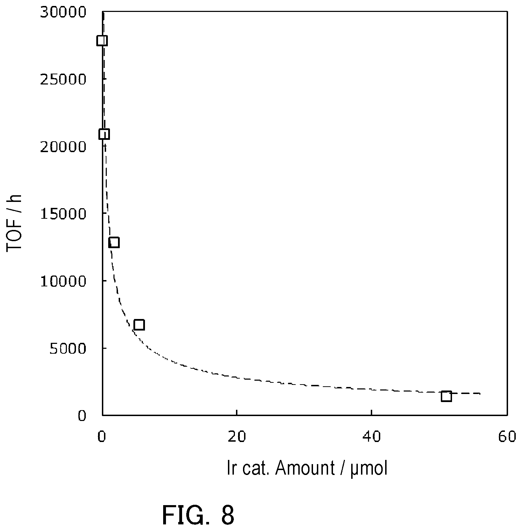

Five types of iridium supporting polymers (Cp*IrBPY/PEI) different in iridium content were obtained in the same manner as in Example 1, except that the amount of polyethyleneimine was set to 1.0 g, the amount of 4,4'-dichloro-2,2'-bipyridine was set to 0.3 g, and the amounts of triaqua(pentamethylcyclopentadienyl)iridium(III) sulfate were set to 0.10 mg (0.22 µmol), 0.50 mg (1.1 µmol), 2.0 mg (4.4 µmol), 5.5 mg (12.1 µmol), and 51.1 mg (112.4 µmol), respectively. FIGS. 7 and 8 illustrate the results of examining the catalytic activity using these iridium supporting polymers as the catalyst.

A method for examining the catalytic activity was the same as that in Example 2.

The results showed that the initial formic acid dehydrogenation reaction rate (initial r on the vertical axis in FIG. 7) increases with an increase in the iridium content (Ir cat. amount on the horizontal axis in FIG. 7) (FIG. 7). On the other hand, the catalyst turnover frequency per mol of iridium (TOF on the vertical axis in FIG. 8) was found to be higher in value the lower the iridium content (Ir cat. amount on the horizontal axis in FIG. 8) (FIG. 8). The conversion rates of formic acid using the catalysts were all 99% or more.

### [Example 8]

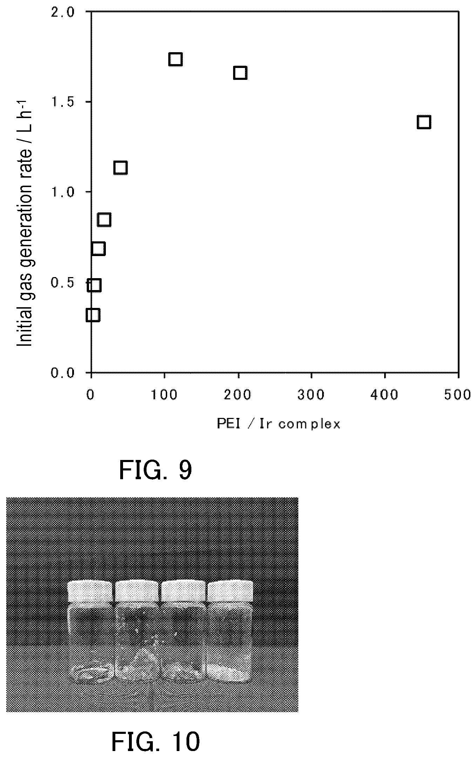

To determine the optimal ratio between polyethyleneimine and iridium, immobilized catalysts changed in the ratio of the polyethyleneimine content to the iridium content were synthesized using the same technique as that in Example 1. Specifically, the immobilized catalysts having the ratios of the polyethyleneimine content to the iridium content of 2, 4, 10, 18, 40, 100, 200, and 450 were synthesized. Then, the initial formic acid dehydrogenation rate was measured using the same technique as that in Example 2. FIG. 9 illustrates the results. The initial formic acid dehydrogenation rate (Initial gas generation rate on the vertical axis in FIG. 9) increased until the value of the ratio of the polyethyleneimine content to the iridium content (PEI/Ir complex on the horizontal axis in FIG. 9) reached 100. However, it was found that, when the value of the ratio of the polyethyleneimine content to the iridium content increased to 200 or 450, the initial formic acid dehydrogenation rate contrarily decreased. When the composition of the generated gas was examined by a gas chromatograph, 50% of hydrogen, 50% carbon dioxide, and less than 1 ppm of carbon monoxide were detected.

### [Example 9: Iridium catalysts supported on various supports]

Next, the results using supports other than various polyethyleneimines are described. First, polyacrylic acid (average molecular weight of 100,000, 100 mg) was added to a ligand (50 mg) represented by Formula (7) below, and then N-(3-dimethylaminopropyl)-N'-ethylcarbodiimide hydrochloride was added, followed by heating to 80°C. The obtained solid was separated by filtration and thoroughly washed with water. Then, 10 mL of water and triaqua(pentamethylcyclopentadienyl)iridium(III) sulfate were added, followed by stirring at 40°C for 3 days under a nitrogen atmosphere. The obtained yellow solid was separated by filtration. The structure of the yellow solid was estimated to be a structure represented by Formula (8) below. This is a polymer compound in which polyacrylic acids are cross-linked with an amide bond.

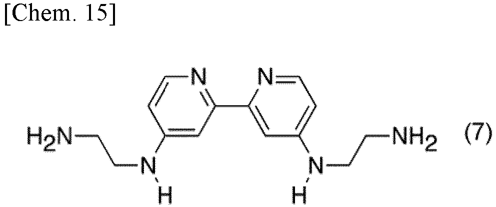

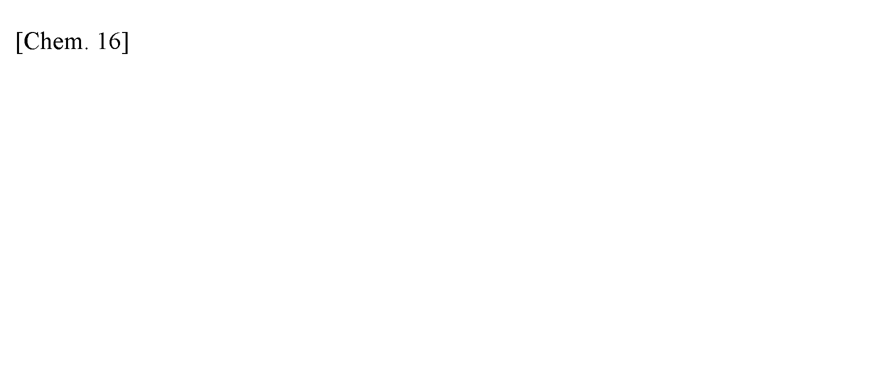

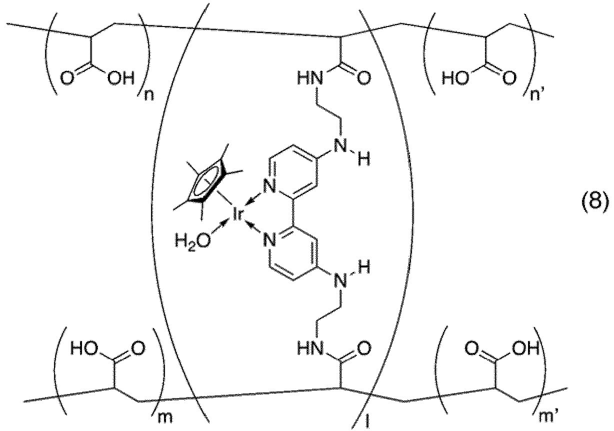

### [Example 10: Iridium catalysts supported on various supports]

Under the same conditions as those in Example 9, a reaction with polyacrylic acid was performed using a ligand represented by Formula (9) below in place of the ligand represented by Formula (7) above, and triaqua(pentamethylcyclopentadienyl)iridium(III) sulfate was added to the obtained solid, followed by stirring at 40°C for 3 days under a nitrogen atmosphere, yielding a yellow solid. The structure of the obtained solid was estimated to be a structure represented by Formula (10) below.

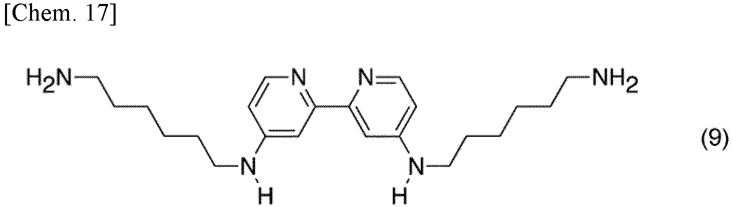

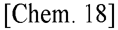

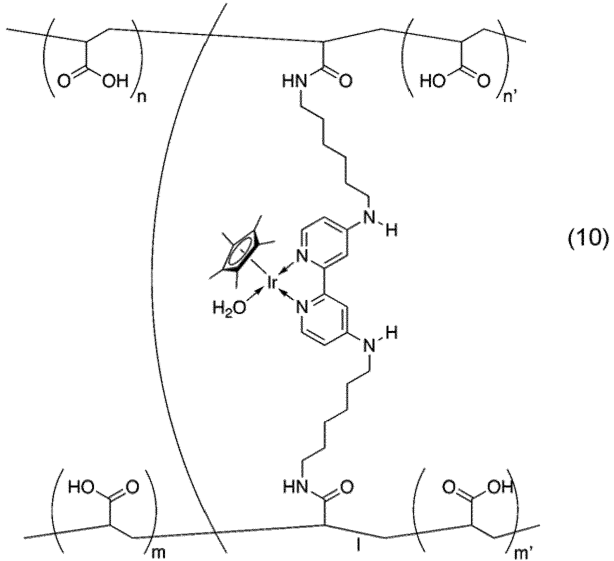

### [Example 11: Formic acid decomposition performance evaluation of immobilized catalyst]

In a 30 mL stainless steel autoclave, a magnetic stirrer and 13.5 mg of the immobilized catalyst represented by Formula (8) above or the immobilized catalyst represented by Formula (10) above were placed, and then 17.5 mL of water and formic acid (98% or more, 2.5 mL) were placed, followed by heating to 80°C in a water bath. The generated gas was measured for the generation rate with a wet gas meter. After the gas generation was completed, formic acid (98% or more, 1 mL) was further added, and the measurement of the generation rate was repeated. The generated gas was collected in a sampling bag, and was measured for the composition by a gas chromatograph.

As the generated gas amount carried out using the above-described technique, about 2.6 L of gas was detected when the immobilized catalyst represented by Formula (8) above was used, and about 2.5 L of gas was detected when the immobilized catalyst represented by Formula (10) above was used. The gas had a composition of 50% by mass of carbon dioxide, 50% by mass of hydrogen, and 1 ppm by mass or less of carbon monoxide. Further, the TOF value, which is the number of formic acid molecules catalyzed by iridium metal per hour, was about 5000 h⁻¹. As a result of repeating the same procedure five times as in Example 2, a decrease in the TOF value was hardly observed.

### [Example 12: Iridium catalysts supported on various supports]

A yellow solid was obtained in the same manner as in Example 9, except for synthesizing polyacrylic acid crosslinked by 0% to 14% using methylene bisacrylamide in place of polyacrylic acid and using the same.

### [Example 13: Iridium catalysts supported on various supports]

A yellow solid was obtained in the same manner as in Example 9, except for synthesizing a copolymer of methacrylic acid and polyacrylic acid in place of polyacrylic acid and using the same.

### [Example 14: Production of hydrogen and carbon dioxide from formic acid at various formic acid concentrations, batch manner]

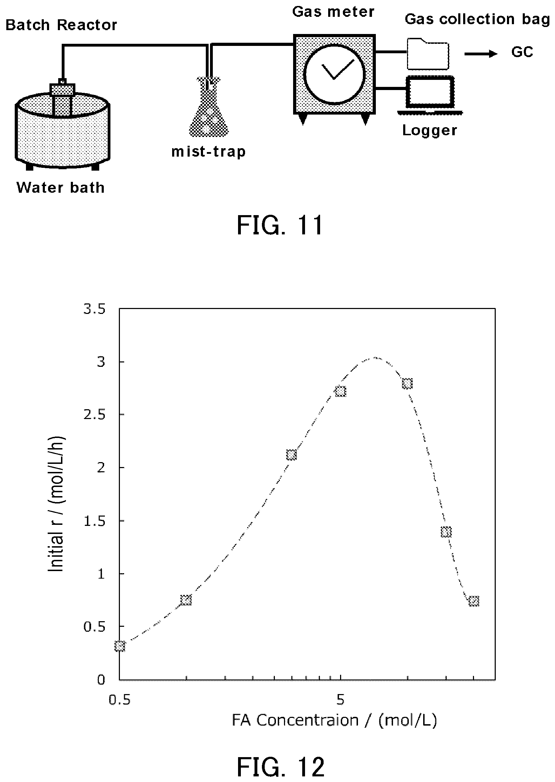

Under the same conditions as those in Example 2, a hydrogen production test using various formic acid concentrations was performed in a batch manner. FIG. 11 illustrates a hydrogen production test method. The formic acid concentrations were set to 0.5 M, 1 M, 2.75 M, 5 M, 10 M, 15 M, and 20 M. Herein, M represents mol/L. As a reactor, a vessel in which a glass inner was inserted into a 50 mL pressure vessel was used. A catalyst (20 mg, iridium content: 33.7 µmol) was placed and 5 mL of formic acid was added at 80°C for reaction. FIG. 12 illustrates the results. The results showed that the efficiency was good at 5 to 10% by mass.

### [Example 15: Production of hydrogen and carbon dioxide from formic acid at various formic acid concentrations, flow manner]

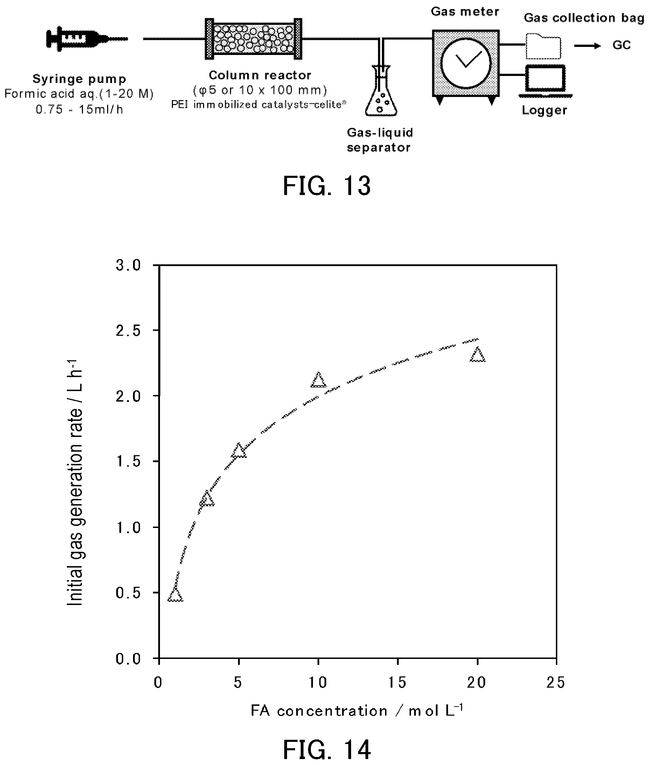

A powder obtained by diluting 256 mg of the catalyst (Cp*IrBPY/PEI) with the same amount of celite was packed into a tubular reactor (10 mm in inner diameter × 100 mm in length), and 5 mL of formic acids of various concentrations was made to flow at a flow rate of 15 mL/h. FIG. 13 illustrates a hydrogen production test method. The formic acid concentrations were set to 1 M, 3 M, 5 M, 10 M, and 20 M. Herein, M represents mol/L. FIG. 14 illustrates the results. When the initial rate of the obtained gas was measured, it was found that the higher the concentration, the higher the generation rate in the flow manner.

### [Comparative Example 1]

In a 30 mL stainless steel autoclave, an iridium complex represented by Formula (11) below or an iridium complex represented by Formula (12) below was placed, and then 17.5 mL of water and formic acid (98% or higher, 2.5 mL) was placed, followed by heating to 80°C in a water bath. The generated gas was measured using a wet gas meter. When the generated gas was collected in a sampling bag and was measured for the composition by a gas chromatograph, no carbon monoxide was detected in both cases where the iridium complexes were used.

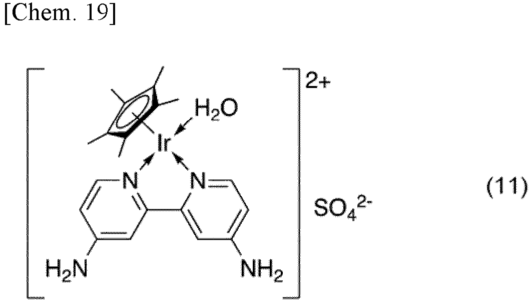

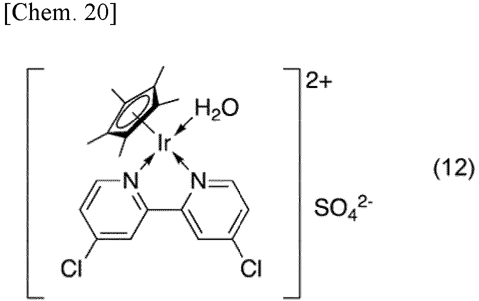

### [Reference Example]

To 1.0 g of polyethyleneimine (branched, Mw up to 2,5000, Mn up to 10,000), 0.05 g, 0.10 g, 0.20 g, and 0.30 g of 4,4'-dichloro-2,2'-bipyridine and water (1.02 mL) were added, followed by heating at 130°C for 24 hours by the same method as that in Example 1. Table 1 shows the results of observing the state of the obtained polymers. Further, FIG. 10 illustrates a photograph of the polymers obtained at that time.

**[Table 1]**

| Polyethyleneimine amount/g | 4,4'-dichloro-2,2'-bipyridine amount/g (mmol) | Obtained polymer/g | State of obtained polymer |
|---|---|---|---|
| 1.0 | 0.05(0.22) | 1.0 | High viscosity liquid |
| 1.0 | 0.10(0.45) | 1.0 | High viscosity gel |
| 1.0 | 0.20(0.89) | 1.2 | Gel |
| 1.0 | 0.30(1.34) | 1.3 | Gel |

Table 1 is a table showing the states of the polymers obtained by crosslinking polyethyleneimines with 4,4'-dichloro-2,2'- bipyridine.

Table 1 proved that, by the addition of about 10% by weight or more of 4,4'-dichloro-2,2'-bipyridine to 1.0 g of polyethyleneimine, the 4,4'-dichloro-2,2'-bipyridine acts as a crosslinking agent and polyethyleneimine can be treated as a gel or a solid.

## Claims

1. A dehydrogenation/carbon dioxide hydrogenation catalyst comprising an iridium complex represented by Formula (3) below which has a ligand bound to a polymer support, 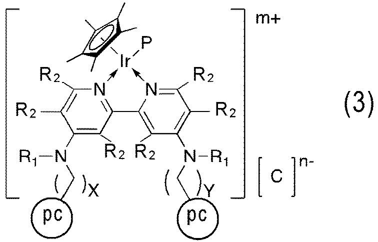 wherein, in Formula (3) above, pc each represents a part of the polymer support, m and n each independently represent a positive integer or 0, X and Y each independently represent a positive integer from 1 to 6, R₁s each independently represent hydrogen or a methyl group, R₂s each independently represent hydrogen or a methyl group, P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).

2. The dehydrogenation/carbon dioxide hydrogenation catalyst according to claim 1, further comprising a polymer support represented by Formula (1) below, 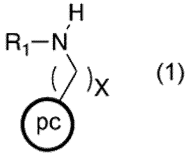 wherein, in Formula (1) above, pc represents a part of the polymer support, X represents a positive integer from 1 to 6, and R₁ each independently represents hydrogen or a methyl group.

3. The dehydrogenation/carbon dioxide hydrogenation catalyst according to claim 1 or 2, further comprising a ligand represented by Formula (2) below bound to a polymer support, 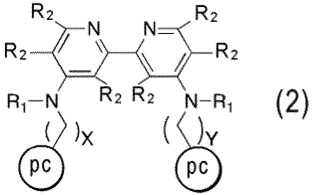 wherein, in Formula (2) above, pc each represents a part of the polymer support, X and Y each independently represent a positive integer from 1 to 6, R₁s each independently represent hydrogen or a methyl group, and R₂s each independently represent hydrogen or a methyl group.

4. The dehydrogenation/carbon dioxide hydrogenation catalyst according to claim 2 or 3, wherein the polymer support represented by Formula (1) above is polyethyleneimine, polystyrene, or a copolymer of ethyleneimine and styrene.

5. A dehydrogenation/carbon dioxide hydrogenation catalyst comprising an iridium complex which has a ligand represented by Formula (6) below bound to a polymer support, 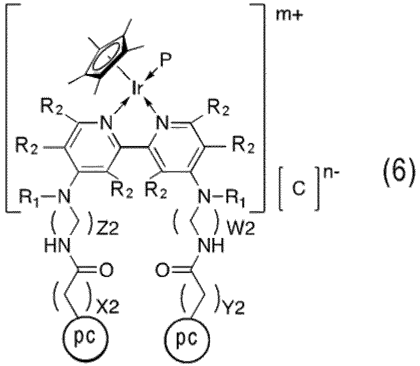 wherein, in Formula (6) above, pc each represents a part of the polymer support, m and n each independently represent a positive integer or 0, X2 and Y2 each independently represent an integer from 0 to 6, Z2 and W2 each independently represent a positive integer from 1 to 6, R₁s each independently represent hydrogen or a methyl group, R₂s each independently represent hydrogen or a methyl group, P represents a molecule or an ion of any one of H₂O, chlorine (Cl), bromine (Br), and acetonitrile (CH₃CN), and C represents any one of a hydroxide ion (OH⁻), a sulfate ion (SO₄²⁻), a chlorine ion (Cl⁻), a bromine ion (Br⁻), a nitrate ion (NO₃⁻), and trifluoromethanesulfonate (CF₃SO₃⁻).

6. The dehydrogenation/carbon dioxide hydrogenation catalyst according to claim 5 further comprising a polymer support represented by Formula (4) below, 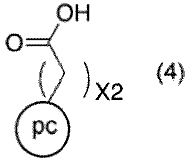 wherein, in Formula (4) above, pc represents a part of the polymer support, and X2 represents an integer from 0 to 6.

7. The dehydrogenation/carbon dioxide hydrogenation catalyst according to claim 5 or 6, further comprising a ligand represented by Formula (5) below bound to a polymer support, 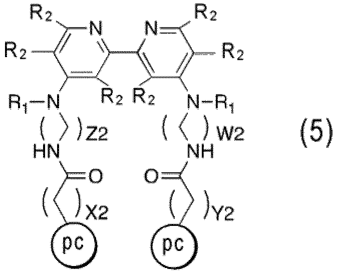 wherein, in Formula (5) above, pc each represents a part of the polymer support, X2 and Y2 each independently represent an integer from 0 to 6, Z2 and W2 each independently represent a positive integer from 1 to 6, R₁s each independently represent hydrogen or a methyl group, and R₂s each independently represent hydrogen or a methyl group.

8. The dehydrogenation/carbon dioxide hydrogenation catalyst according to claim 6 or 7, wherein the polymer support represented by Formula (4) above is polyacrylic acid, polymethacrylic acid, polymaleic acid, or a copolymer of at least two selected from the group consisting of acrylic acid, methacrylic acid, and maleic acid.

9. A hydrogen gas production method comprising generating a gas containing hydrogen and carbon dioxide and having a total pressure of 0.1 MPa or more and 200 MPa or less from a solution containing formic acid,
wherein the catalyst according to any one of claims 1 to 8 is used.

10. A carbon dioxide hydrogenation method comprising generating formic acid or formate from a gas containing hydrogen and carbon dioxide and having a total pressure of 0.1 MPa or more and 200 MPa or less,
wherein the catalyst according to any one of claims 1 to 8 is used.
